# EUROPÄISCHE PATENTSCHRIFT

(11) **EP 1 539 267 B1**
(45) Veröffentlichungstag und Bekanntmachung des Hinweises auf die Patenterteilung: **31.08.2016**
(21) Anmeldenummer: 03750586.4
(22) Anmeldetag: 19.09.2003
(51) Int. Cl.: A61L 29/16, A61L 31/16

(54) **MEDIZINISCHE VORRICHTUNG ZUR ARZNEIMITTELABGABE**
MEDICAL DEVICE FOR DISPENSING MEDICAMENTS
DISPOSITIF MEDICAL POUR L'ADMINISTRATION DE MEDICAMENTS

(30) Priorität: 20.09.2002 DE 10244847
(43) Veröffentlichungstag der Anmeldung: 15.06.2005
(73) Patentinhaber: Bayer Intellectual Property GmbH, 40789 Monheim (DE)
(72) Erfinder: SPECK, Ulrich, 13465 Berlin (DE); SCHELLER, Bruno, 66111 Saarbrücken (DE); SCHEURER, Hartmut, P., 82031 Grünwald (DE)
(74) Vertreter: Zimmermann & Partner Patentanwälte mbB
(86) Internationale Anmeldenummer: PCT/EP2003/010480
(87) Internationale Veröffentlichungsnummer: WO 2004/028610

(56) Entgegenhaltungen:
- EP-A- 0 829 238
- WO-A-00/44414
- WO-A-00/45744
- WO-A-92/11890
- WO-A-92/15282
- DE-C- 4 225 553
- US-A- 5 320 634
- US-A- 5 370 614
- US-A- 5 571 086
- US-A- 5 626 562
- US-A- 5 792 158
- US-A1- 2002 123 505
- US-B1- 6 306 151

## Beschreibung

Die Erfindung betrifft eine medizinische Vorrichtung zur Arzneimittelabgabe für die selektive Therapie bestimmter Gewebeabschnitte oder Organteile und ein Verfahren zur Herstellung derartiger arzneimittelbeschichteter Geräte.

Zahlreiche Erkrankungen betreffen nicht den gesamten Organismus gleichzeitig sondern sind auf bestimmte Gewebearten, häufig auch auf sehr begrenzte einzelne Gewebebezirke oder Organteile beschränkt. Beispiele dafür finden sich unter den Tumor-, Gelenk- und Gefäßerkrankungen.

Die Pharmakotherapie auch dieser Erkrankungen erfolgt im allgemeinen durch orale oder intravenöse Gabe von Arzneimittelstoffen, die sich im ganzen Körper verteilen und in vielen Fällen gerade bei schweren Erkrankungen unerwünschte Wirkungen in gesunden Geweben und Organen verursachen, die die therapeutische Anwendung begrenzen. Eine selektive Therapie der erkrankten Gewebe wurde mittels spezifisch an erkranktes Gewebe bindende Arzneistoffe (z. B. Antikörper) unter Beibehaltung des Applikationsweges oder durch selektive Verabreichung, z. B. durch direkte Injektion in das erkrankte Gewebe oder durch Zufuhr über Katheter zu den das erkrankte Gewebe versorgenden Blutgefäßen, erreicht. Im Falle der selektiven Verabreichung entstehen durch die meist kurze Wirkdauer der Arzneistoffe und die invasiven Verabreichungswege Probleme, da sich eine beliebig wiederholte Gabe verbietet. Bei selektiver Verabreichung über den das erkrankte Gewebe versorgenden Blutstrom ergibt sich das zusätzliche Problem ungenügender Extraktion der Arzneistoffe bei der raschen Passage des Blutes oder der Wirkstofflösung durch die Blutgefäße.

Diesen Problemen wurde bisher durch unterschiedliche pharmazeutische Präparate mit verzögerter Wirkstofffreigabe, arzneimittelfreisetzende Implantate oder für längere Zeit funktionsfähige selektive Zugangswege wie implantierte Katheter etc. begegnet.

Es ist bereits bekannt, die Oberfläche von in den Körper eingebrachten medizinischen Geräten, insbesondere Kathetern, mit Mitteln zur Verbesserung der Gleitfähigkeit oder zur Verhinderung der Blutgerinnung, jedoch ohne therapeutische Wirkung, zu beschichten.

Darüber hinaus werden Katheter mit speziellen Vorrichtungen versehen, um Arzneimittel in die Arterienwand zu injizieren, beispielsweise mittels Nadeln oder hohem Injektionsdruck über eine an der Gefäßwand anliegende Perforation der Katheterwand.

Andere Prinzipien beruhen darauf, die Kontaktzeit zwischen Arterienwand und einer über den Katheter applizierten Wirkstoffzubereitung zu verlängern, indem entweder der Blutstrom für einen entsprechenden Zeitraum unterbunden wird, z. B. Doppelballonkatheter mit zwischen den Ballons befindlicher mit der Arzneistofflösung gefüllter Kammer oder Hohlräumen zwischen der z. B. mit Wülsten versehenen Ballonaußenwand, wobei der Blutstrom durch einen den Ballon passierenden Kanal in begrenztem Maße aufrechterhalten werden kann.

Gemäß der US 5 102 402 werden Arzneistoffe in Form von Mikrokapseln zur verzögerten Wirkstofffreigabe lose in vorgeformten Vertiefungen von Ballonkathetern untergebracht. Nach Expansion des Ballons sollen die Mikrokapseln in die Gefäßwand gedrückt werden, dort verbleiben und den oder die Wirkstoffe langsam freisetzen. Zahlreiche Autoren schlagen auch vor, Arzneistoffe in Hydrogel eingebettet auf Ballonkatheter aufzubringen, wobei die Funktion des Hydrogels als Haftmittel, zur Verbesserung der Gleitfähigkeit oder Verzögerung der Freisetzung der Arzneistoffe offen bleibt.

Nachteil der genannten Produkte ist in jedem Falle der komplexe Aufbau mit entsprechenden Problemen bei Herstellung, Qualitätskontrolle und Kosten sowie zusätzlichen, Arzt und Patient belastenden Arbeitsschritten bei der Anwendung. Ein Teil der genannten Methoden führt zu einer über die beabsichtigte Gefäßerweiterung hinausgehenden gänzlich unerwünschten Gefäßverletzung. Andererseits hat jede zur Verlängerung der Kontaktzeit vorgesehene Maßnahme eine zusätzliche Minderversorgung der nachgeschalteten Gewebe im Blut und Sauerstoff zur Folge.

Der Vollständigkeit halber wird noch auf eine in der WO 01/24866 beschriebenen Vorrichtung zum Verhindern der Restenose verwiesen, die mit einer von natürlichen Zellmembranen abgeleiteten lipiden Ceramide-Substanz beschichtet sind. Diese Substanz wird aufgrund ihrer bei gebräuchlichen Arzneistoffen nicht anzutreffenden Affinität zu den Zellwänden der Arterienwand verwendet. In der Fachliteratur wird jedoch weiterhin die Auffassung vertreten, dass eine Arzneimittelprophylaxe der Restenose eine Freisetzung der erforderlichen Wirkstoffe über Tage erfordert.

Dokument US 5,792,158 betrifft einen Dilatator mit expandierbaren Schneiden, wobei die Vorrichtung ein Dilatatorgehäuse und mehrere Klingen umfasst, und wobei das Gehäuse derart ausgebildet ist, dass es eine Kammer umgibt und wobei die Kammer einen inflatierbaren Ballon enthält.

Dokument US 5,370,614 betrifft ein Verfahren zum Herstellen eines Arzneistoff abgebenden Ballonkatheters.

Der Erfindung liegt die Aufgabe zugrunde, eine Vorrichtung für eine auf bestimmte Gewebebereiche oder Organteile begrenzte Arzneimittelabgabe bereitzustellen, die ohne schädigenden Einfluss auf gesundes Gewebe eine starke therapeutische Wirkung ausübt, den Patienten nur wenig belastet und mit geringem Aufwand angewendet und hergestellt werden kann.

Erfindungsgemäß wird die Aufgabe mit einer gemäß den Merkmalen der Ansprüche 1 und 22 ausgebildeten bzw. hergestellten Vorrichtung gelöst. Aus den Unteransprüchen ergeben sich weitere Merkmale und vorteilhafte Weiterbildungen der Erfindung.

Durch die Erfindung werden in einem einfachen Herstellungsverfahren verbesserte arzneistofftragende Ballonkatheter oder ähnliche medizinische Vorrichtungen bereitgestellt, die vielseitig anwendbar sind und eine sofortige Wirkstofffreigabe ermöglichen. Überraschend und entgegen der Lehrmeinung ist keine andauernde Wirkstofffreisetzung aus einer inerten Matrix (Polymer, Hydrogel, Mikrokapseln etc.) oder speziellen chemischen oder physikalischen Zuständen der Wirkstoffe erforderlich oder nützlich. Daher werden auch keine aufwendigen Techniken zur Produktion oder Kontrolle von Depotformulierungen benötigt. In vielen Fällen behindern Gefäßverengungen das Einbringen des Ballons in den Bereich des zu behandelnden Gewebeabschnitts. Auch die erkrankten Gewebeabschnitte oder Organteile weisen oftmals krankhafte Verengungen auf. Um die Platzierung des oder der arzneistofftragenden Ballone innerhalb der entsprechenden Körperhöhlen im Bereich des zu therapierenden Gewebeabschnitts oder Organteils zu erleichtern wird erfindungsgemäß ein Ballonkatheter verwendet, der mindestens eine aus mindestens einem Ballon herausragende oder auf der Oberfläche des Ballons aufsitzende Vorrichtung zur Schlitzung von Stenosen zumindest im Bereich der erkrankten Gewebsabschnitte oder Organteile aufweist. Die Vorrichtung zur Schlitzung von Stenosen kann dabei aus mindestens einer drahtartigen Vorrichtung bestehen, wobei die drahtartige Vorrichtung parallel zur Längsachse des Ballons angeordnet ist. Insbesondere ist die Vorrichtung aus mindestens zwei drahtartigen Vorrichtungen und bildet eine gitterartige Konstruktion bildet, wobei die Längsachse der gitterartigen Konstruktion wiederum parallel oder achsparallel zur Längsachse des Ballons angeordnet ist. Es ist aber auch möglich, dass die Vorrichtung zur Schlitzung von Stenosen aus mindestens einer klingenartigen Vorrichtung oder aus mindestens einem aus dem Ballon herausragenden oder auf der Oberfläche des Ballons aufsitzenden Vorsprung besteht. Als Materialen für die Herstellung der klingenartige Vorrichtung eignen sich Metalle, Metall-Legierungen, Kunststoffe oder Kombinationen davon. Insbesondere können auch sogenannte Formgedächtnislegierungen Verwendung finden. Durch die Ausbildung einer Vorrichtung zur Schlitzung von Stenosen können vorteilhafterweise zum Beispiel kalzifizierte Gefäßverengungen oder In-Stent-Restenosen geschlitzt werden, wodurch die Elastizität der so behandelten Gewebeabschnitte deutlich erhöht wird und zudem die Zugänglichkeit für die Arzneistoffe verbessert wird.

Die Beschichtung von auf Kathetern befindlichen Ballonen mit Arzneistoffen gemäß der vorliegenden Erfindung ist insofern von besonderem Nutzen als nach dem Erweitern von Blutgefäßen oder anderen Hohlräumen im Körper mit den Ballonen häufig der Bedarf an therapeutischen Maßnahmen besteht, um eine Verengung oder einen Verschluss des mit dem Ballon unter Druck geschaffenen Lumens zu verhindern, Tumorwachstum zu begrenzen oder Heilungsprozesse einschließlich der Bildung von Kollateralkreisläufen zu fördern. Dies kann durch Arzneistoffe erreicht werden, die ihre Wirkung in unmittelbarer Nähe der Ballonoberfläche entfalten. Die Arzneistoffe haften auf dem Weg zum Ziel - meist durch intensiv durchblutete Arterien - bis zur Entfaltung des Ballons fest auf diesem, werden dann während der kurzen, oft nur Sekunden andauernden Kontaktzeit des entfalteten Ballons an das Gewebe in wirksamer Dosis abgegeben und von diesem in einer Weise aufgenommen, die das Abspülen durch den nach Deflation des Ballons sofort wieder einsetzenden Blutstrom vermeidet.

Zur Beschichtung können erfindungsgemäß zusätzlich Drähte wie sie zur Führung von Kathetern verwendet werden, Nadeln und Katheter bzw. Teile von Kathetern, die zumindest für kurze Zeit mit Druck gegen erkranktes Gewebe gepresst werden, vorgesehen. Bevorzugte Kathetermaterialien sind Polyamide, Polyamid-Gemische und Copolymere, Polyethylenterephthalat, Polyethylin und Copolymere, Polyurethan, Naturkautschuk und dessen Derivate. Die Länge und der Durchmesser der für die Pharmakotherapie vorgesehenen Bereiche der Katheter bzw. Ballone ist für die Anwendung nicht von entscheidender Bedeutung, da die Dosierung in µg Wirkstoff/mm² Oberfläche berechnet wird. Beispielsweise sind für die Koronardilatationen Ballone im Bereich von 2 - 4 mm Durchmesser und von 1,0 - 4,0 cm Länge gebräuchlich. Für andere Gefäße können auch Ballone bis zu > 20 mm Durchmesser und Längen bis zu > 10 cm eingesetzt werden. Die zu beschichtenden Oberflächen können glatt (d. h. ohne besondere Struktur zur Aufnahme der Wirkstoffe), aufgeraut oder in beliebiger Weise mit Strukturen versehen sein, wobei spezielle Oberflächenstrukturen nicht Voraussetzung für die Haftung der Wirkstoffe sind, die Haftung aber auch nicht behindern. Die Haftung der Wirkstoffe auf den Ballonoberflächen wird ausschließlich durch die Wahl geeigneter Lösungsmittel und ggf. die Haftung beeinflussende Zusatzstoffe bewirkt. Sie ist selbst auf äußerlich vollständig glatten Ballonoberflächen überraschend fest.

Alle Oberflächen können zusätzlich mit Substanzen beschichtet worden sein oder werden, die die Gleitfähigkeit der Produkte verbessern, die Gerinnung von Blut an der Oberfläche verhindern oder sonstige Eigenschaften der Medizinprodukte verbessern, ohne dass die zur Beschichtung benutzten Materialien an die Umgebung abgegeben werden müssen und ohne dass die Beschichten die Abgabe der Wirkstoffe zur Behandlung der Zielgewebe und damit die Wirksamkeit wesentlich einschränkt.

Ballonkatheter werden aus sehr dünnen Kunststoffschläuchen durch Aufweitung eines Segments von 1 bis ca. 10 cm Länge geformt. Die aufgeweitete, sehr dünnwandige Ballonmembran wird anschließend in mehrere längs zur Katheterachse angeordnete Falten gelegt und fest um die Katheterachse gewickelt, so dass der aufgeweitete Bereich im gefalteten Zustand einen nur minimal größeren Durchmesser aufweist als der übrige Katheter. Die enge Faltung der Ballonhülle ist Voraussetzung für die problemlose Passage des Ballonkatheters durch Einführungsschleusen, Führungskatheter und z. B. stark verengte Abschnitte von Blutgefäßen.

Die Ballone von Kathetern können in gefaltetem und entfaltetem Zustand beschichtet werden, wobei in jedem Fall eine intakte, ausreichend gleichmäßige Beschichtung der Oberfläche erzielt wird und die Wirkstoffe auch während des Einfaltens eines im entfalteten Zustand beschichteten Ballonkatheters ausreichend fest an dessen Oberfläche haften.

Die Herstellung eines in entfaltetem Zustand beschichteten Ballons erfolgt ohne Beeinträchtigung der Beschichtung beispielsweise durch die Verwendung von Ballonhüllen mit vorgeformten Falten und Biegungen, deren Struktur im Material durch das Aufdehnen nicht verloren geht und die nach Ablassen des Druckes aus dem Ballon bewirken, dass sich die Ballonhülle wieder regelrecht wenigstens lose einfaltet, ohne dass es einer äußeren Kraft als primäre Ursache bedarf. Erst danach werden die vorgeformten Falten von außen oder durch Vakuum zusammengepresst. In keinem Falle sind Falten zum Festhalten des Wirkstoffs erforderlich. Weiterhin kann die Einfaltung durch geringe mechanische Kräfte mittels sehr glatter Materialien bewirkt werden, wobei die Werkzeuge auch beispielsweise mit schlüpfrigen biokompatiblen Flüssigkeiten benetzt sein können, in denen sich die Wirkstoffe nicht oder jedenfalls nicht gut lösen.

Gemäß einer weiteren Erfindungsvariante werden die Ballone fertig gefalteter Ballonkatheter durch Tauchen in niedrig viskose Wirkstofflösungen beschichtet. Dabei dringen Lösungsmittel und Wirkstoff zwischen die extrem engen Falten und bilden dort einen überraschend gleichmäßigen und in der Dosis reproduzierbaren Belag, der durch keinen weiteren Arbeitsschritt beschädigt wird. Die außen anhaftende Lösung bzw. der nach Trocknen des Lösungsmittels außen anhaftende Belag kann dort belassen oder in einem weiteren Arbeitsschritt entfernt werden, so dass nur der von den Falten des Ballons verdeckte Wirkstoff erhalten bleibt.

Nach der Beschichtung kann bei gefaltetem Ballon ein Stent auf den Ballonkatheter geschoben und auf diesem festgepresst werden. Danach ist nur noch die Sterilisation, z. B. mittels Ethylenoxid, erforderlich.

Der so gestaltete Arbeitsgang ist außerordentlich einfach, wenig störanfällig und auch mit mechanisch, chemisch und physikalisch empfindlichen Beschichtungsmaterialien durchzuführen. Es hat sich gezeigt, dass die Beschichtung nach diesem Verfahren zu keiner unerwünschten Lockerung oder Verklebung der Faltung führt und dass der derart aufgetragene Wirkstoff fest genug haftet, um auf dem Weg durch das Blut nicht abgetragen zu werden, andererseits bei Inflation des Ballons im Zielgewebe den Wirkstoff weitgehend freigibt.

Als Arzneistoffe kommen stark lipophile, weitgehend wasserunlösliche an beliebige Gewebebestandteile bindende stark wirksame Arzneistoffe in Frage. Als lipophil werden Arzneistoffe bezeichnet, deren Verteilungskoeffizient Butanol: wässriger Puffer pH 7 ≥ 0.5, bevorzugt ≥ 1 und besonders bevorzugt ≥ 5 bzw. Ocatanol: wässriger Puffer pH 7 ≥ 1, bevorzugt ≥ 10, besonders bevorzugt > 50 ist. Alternativ oder zusätzlich sollen die Arzneistoffe zu > 10 %, bevorzugt zu > 50 %, besonders bevorzugt zu > 80 % reversibel und/oder irreversibel an Zellbestandteile binden. Bevorzugt sind Substanzen zur Hemmung der Zellproliferation oder auch entzündlicher Prozesse oder Antioxidantien wie Paclitaxel und andere Taxane, Rapamycin und verwandte Substanzen, Tacrolimus und verwandte Substanzen, Corticoide, Sexualhormone (Östrogen, Estradiol, Antiandrogene) und verwandte Substanzen, Statine, Epothilone, Probucol, Prostacycline, Angiogeneseinduktoren etc..

Die Substanzen liegen bevorzugt als trockene Festsubstanz oder als Öl auf den Oberflächen der unterschiedlichen Medizinprodukte vor. Bevorzugt sind Partikel geringster Größe (in der Mehrzahl > 5µm, bevorzugt > 1 µm, besonders bevorzugt > 0.1 µm), besonders bevorzugt sind amorphe, nicht kristalline Strukturen feinster Partikelgröße, die bei Kontakt mit Gewebe wegen ihrer großen Oberfläche trotz grundsätzlich geringer Wasserlöslichkeit der Arzneistoffe rasch in Lösung gehen und nicht als Mikrokapseln fungieren, d. h. sich spontan und schnell lösen. Dabei ist es ausreichend, dass eine wirksame Dosis in Form kleinster oder amorpher Partikel vorliegt; größere Partikel tragen zwar zur Wirkstoffkonzentration im Gewebe kaum bei, stören aber auch nicht. Die Dosierung richtet sich nach der erwünschten Wirkung und der Wirksamkeit des verwendeten Arzneistoffes. Sie kann bis zu 5 µg/mm² erreichen, wobei dies keine Obergrenze darstellt. Geringere Dosierungen sind leichter zu realisieren.

Eine gute Haftung an den Oberflächen der Katheter, Nadeln oder Drähte bei Verbesserung der Aufnahme in die Gewebe wird durch Einbetten von stark lipophilen, schlecht wasserlöslichen Wirkstoffen in eine leicht wasserlösliche Matrixsubstanz erreicht. Als Matrixsubstanz sind niedermolekulare (Molekulargewicht < 5000 D, bevorzugt < 2000 D) hydrophile Substanzen wie in vivo verwendete Kontrastmittel und Farbstoffe für unterschiedliche diagnostische Verfahren in der Medizin, Zucker und verwandte Substanzen wie Zuckeralkohole, niedermolekulare Polyethylenglycole, biokompatible organische und anorganische Salze wie z. B. Benzoate, Salze und andere Derivate der Salicylsäure etc. geeignet. Als Kontrastmittel sei auf die jodierten Röntgenkontrastmittel und die paramagnetischen Chelate verwiesen, Beispiele für Farbstoffe sind Indocyaningrün, Fluorescein und Methylenblau. Hilfsstoffe können auch zur Verbesserung der Lagerfähigkeit der Produkte dienen, spezielle ergänzende pharmakologische Wirkungen verursachen oder der Qualitätskontrolle dienen.

In einer weiteren Ausführung können die pharmazeutischen Wirkstoffe an Partikel adsorbiert oder mit niedermolekularer Matrix auf die Oberflächen geeigneter Medizinprodukte aufgebracht werden. Als Partikel sind wiederum als biokompatibel bekannte Diagnostika wie Ferrite und diverse Kontrastmittel für die Sonographie geeignet.

Hilfsstoffe aller Art können in geringerer oder höherer Dosis als die Wirkstoffe eingesetzt werden.

Die Beschichtung der Medizinprodukte erfolgt mittels Lösungen, Suspensionen oder Emulsionen der genannten Arzneistoffe und Hilfsstoffe. Geeignete Lösungs-, Suspendier- oder Emulsionsmedien sind beispielsweise Ethanol, Isopropanol, Ethylacetat, Diethylether, Aceton, Dimethylsulfoxid, Dimethylformamid, Glycerin, Wasser oder Mischungen derselben. Die Auswahl der Lösungsmittel folgt entsprechend der Löslichkeit der Wirkstoffe und Zusatzstoffe sowie der Benetzung der zu beschichtenden Oberflächen und der Wirkung auf die Struktur der nach Verdampfen der Lösungsmittel zurückbleibenden Beschichtung und Partikel, deren Haftung auf der Oberfläche und die Wirkstoffobertragung in das Gewebe während sehr kurzer Kontaktzeiten.

Die Auftragung kann beispielsweise durch Tauchen, Bestreichen, Auftragen mittels Volumenmesseinrichtungen oder Besprühen jeweils bei unterschiedlichen Temperaturen und ggf. Dampfsättigungen der Lösungsmittel in der Atmosphäre geschehen. Der Vorgang kann mehrfach, ggf. auch unter Verwendung verschiedener Lösungsmittel und Hilfsstoffe, wiederholt werden.

Die Ballone fertig gefalteter Ballonkatheter lassen sich durch Tauchen in wirkstoffhaltige Lösungen oder andere Maßnahmen erstaunlich gleichmäßig, reproduzierbar, in der Dosis steuerbar und ohne Beeinträchtigung der Funktion der Katheter beschichten. Bei wiederholtem Tauchen in ungesättigten Wirkstofflösungen kommt es wider Erwarten nicht zu einer vollständigen Ablösung des vorher aufgetragenen Wirkstoffs sondern zu einer reproduzierbaren Erhöhung des Wirkstoffgehaltes der Ballone.

Überschüssige Lösung bzw. überschüssige, außen lose anhaftende Substanzen aus der Beschichtungslösung können mit einfachen Methoden entfernt werden, ohne dass es zu einer Beeinträchtigung der Wirksamkeit der Beschichtung kommt.

Die erfindungsgemäß ausgebildeten und hergestellten medizinischen Vorrichtungen unterschiedlicher Art kommen kurzzeitig, d. h. für Sekunden, Minuten oder wenige Stunden mit dem Gewebe in Kontakt. In einigen Fällen ist es erwünscht, das Gewebe in unmittelbarer Nähe des Medizinproduktes pharmakologisch zu behandeln, zum Beispiel an überschießendem Wachstum als Reaktion auf eine Verletzung zu hindern oder Tumorwachstum zu reduzieren oder die Einsprossung von Blutgefäßen zu fördern oder Entzündungsreaktionen zu vermindern. In all diesen Fällen kann mit dem oben beschriebenen Verfahren eine hohe lokale Arzneistoffkonzentration für erstaunlich lange Zeit erzielt werden. Ein wesentlicher Vorteil ist die außerordentliche Vielfalt der Einsatzmöglichkeiten der beschriebenen Produkte und Verfahren.

Eine bevorzugte Anwendung ist die Verminderung der durch die Gefäßdilatation mittels Ballonkathetern induzierte Hyperproliferation der Gefäßwände. Diese ist im Bereich gegebenenfalls implantierter Gefäßstützen (Stents) auch durch Beschichtung der Stents mit Arzneistoffen zu erzielen, allerdings nur im unmittelbar durch den Stent bedeckten Gefäßbereich. Die beschichteten Ballonkatheter behandeln darüber hinaus die behandlungsbedürftigen Bereiche kurz vor und kurz hinter dem Stent, sie können ohne erneute Stentimplantation den Bereich innerhalb bereits vorhandener Stents behandeln oder Gefäße, in die kein Stent implantiert werden soll oder kann. Vorteilhaft gegenüber den über einen langen Zeitraum Arzneistoff freisetzenden Stents ist die bessere Einheilung bei gleichzeitig guter Hemmung der Hyperproliferation und das geringere Thromboserisiko.

Nachfolgend werden mehrere Ausführungsformen der Erfindung am Beispiel der Beschichtung von Ballonkathetern sowie in Bezug auf die Haftung der Beschichtung im Blut, die Restenosehemmung und den Wirkstoffgehalt der Katheter beschrieben.

Beispiel 1:

Beschichten eines expandierten Ballonkatheters mit Paclitaxel in Ethylacetat

Ballonkatheter der Fa. BMT, Oberpfaffenhofen/München, Deutschland, mit der Bezeichnung Joker Lite, Ballongröße 2.5 mm x 20 mm, werden nach maximaler Expansion für 1 min. über die gesamte Ballonlänge in Ethylacetat, 18.8 mg Paclitaxel/ml, + 1 % pharmazeutisches Olivenöl, getaucht, getrocknet: Paclitaxelgehalt 39 µg (nach Extraktion mit Ethanol, HPLC).

### Beispiels 2:

Beschichten eines gefalteten Ballonkatheters mit Paclitaxel in Ethylacetat

Ballonkatheter der Fa. BMT, Oberpfaffenhofen/München, Deutschland, mit der Bezeichnung Joker Lite, Ballongröße 2.5 mm x 20 mm, werden im gefalteten Zustand für 1 min. über die gesamte Ballonlänge in Ethylacetat, 18.8 mg Paclitaxel/ml, + 1 % pharmazeutisches Olivenöl, getaucht, getrocknet: Paclitaxelgehalt 69 µg

### Beispiel 3:

Beschichten eines gefalteten Ballonkatheters mit Paclitaxel in Ethylacetat
a) Ballonkatheter der Fa. BMT, Oberpfaffenhofen/München, Deutschland, mit der Bezeichnung Joker Lite, Ballongröße2.5 mm x 20 mm, werden im gefalteten Zustand für 1 min. über die gesamte Ballonlänge in Ethylacetat, 16.6 mg Paclitaxel/ml, getaucht, 4 h getrocknet: Paclitaxelgehalt 54 µg
b) Ebenso, jedoch noch 2 mal 5 sec. mit 1 h Trocknungszeit nach jeden Tauchvorgang in Lösung A (= 3.33 ml Ethylacetat + 100.0 mg Paclitaxel) getaucht: Paclitaxelgehalt 126 µg
c) Ebenso, jedoch noch 4 mal 5 sec. mit 1 h Trocknungszeit nach jedem Tauchvorgang in die gleiche Lösung getaucht: Paclitaxelgehalt 158 µg

### Beispiel 4:

### Beschichten eines Ballonkatheters mit Paclitaxel in Aceton

350 mg Paclitaxel in 9.0 ml Aceton lösen; Ballonkatheter der Fa. BMT, Oberpfaffenhofen/München, Deutschland, mit der Bezeichnung Joker Lite, Ballongröße 2.5 mm x 20 mm, werden im maximal expandierten Zustand für 1 min. über die gesamte Ballonlänge getaucht, entnommen, das Lösungsmittel wird bei Raumtemperatur 12 h getrocknet. Danach wird der Ballon deflatiert und mit PTFE-beschichtetem Werkzeug in üblicher Weise eingefaltet. Optional kann ein Stent geeigneter Größe auf den Ballon gecrimpt werden: 29 µg Paclitaxel auf dem Ballon.

### Beispiel 5:

### Beschichten eines Ballonkatheters mit Paclitaxel in Aceton

a) Tauchen gefaltete Ballonkatheter der Fa. BMT mit der Bezeichnung Allegro, Ballongröße 2.5 x 20 mm in ein Gemisch von 0.15 ml Ethanol + 4.5 µl Ultravist 300 (Röntgenkontrastmittel der Schering AG, Berlin, Deutschland) + 1.35 ml Aceton + 0.8 mg Sudanrot + 30.0 mg Paclitaxel:
   Die gefalteten Ballonabschnitte der Katheter werden 5 x getaucht, das 1. Mal für 1 Minute, dann 3 h Trocknungszeit, danach 4 mal für je 5 sec. im Abstand von 1 h; danach wurde ein Stent aufgecrimpt und der Katheter mit Stent in üblicher Weise mit Ethylenoxid sterilisiert:
      Paclitaxelgehalt 172 µg, keine mittels HPLC detektierbaren Zersetzungsprodukte des Wirkstoffs
b) Statt Ultravist 300 wird eine gesättigte wässrige Mannit-Lösung zugesetzt.
c) Statt Ultravist 300 wird eine gesättigte wässrige Natrium-Salicylat-Lösung, pH 7.5, zugesetzt.
d) Der fertigen Lösung nach (5a) werden 5 mg Acethylsalicylsäure zugesetzt.
e) Der fertigen Lösung nach (5a) werden 5 mg Glycerin zugesetzt.

### Beispiel 6:

### Haftung des Wirkstoffs in Blut

Es wurden 12 Ballonkatheter der Fa. BMT mit der Bezeichnung Allegro, Ballongröße 2.5 x 20 mm, verwendet. Je 6 Stück der gefalteten Ballonabschnitte der Katheter wurden entweder in [0.15 ml Ethanol + 4.5 µl Ultravist 300 + 1.35 ml Aceton + 0.8 mg Sudanrot + 30.0 mg Paclitaxel] oder in [1.5 ml Ethylacetat + 0.8 mg Sudanrot + 31.0 mg Paclitaxel] 5 x getaucht, das 1. Mal für 1 Minute, dann 3 h Trocknungszeit, danach 4 mal für je 5 sec. im Abstand von 1 h; danach wurden je 3 gefaltete Ballone jeder Serie in 50 ml Humanblut 5 min. bei 37 °C leicht bewegt und dann zur Analyse des Paclitaxel-Gehaltes entnommen:
Verminderung der Mittelwerte (n=3 je Beschichtungsmethode) durch 5 Minuten Bewegung in Blut im Vergleich zu je 3 Kontrollkathetern, die nicht in Blut inkubiert wurden.
   Aceton: 12%
   Ethylacetat: 10%

### Beispiel 7:

Untersuchung der Restenosehemmung nach Angioplastie und Stentimplantation an den Koronarien von Schweinen
Gefaltete Ballonkatheter der Fa. BMT Typ Joker Lite, MBT 3.5 x 20 mm oder 3.0 x 20 mm wurden entweder in
Lösung A) 3.33 ml Ethylacetat (EA) + 100.0 mg Paclitaxel oder in
Lösung B) 0.45 ml Ethanol + 100 µl Ultravist-370 + 4.5 ml Aceton (Ac) + 150.0 mg Paclitaxel
für 1 min. eingetaucht und bei Raumtemperatur über Nacht getrocknet. Ein weiterer (niedrige Dosis = L) bzw. 4 weitere (hohe Dosis = H) Tauchvorgänge wurden jeweils nur für 5 Sekunden am nächsten Tag im Abstand von 1 h durchgeführt.
Wirkstoffgehalt nach 2 maligem Tauchen in Lösung (B) im Mittel 250 µg, bei 5 maligem Tauchen in Lösung (B) 500 µg, in Lösung (A) 400 µg.
Insgesamt 22 Schweinen wurde mittels der mit Paclitaxel beschichteten Katheter oder mittels unbeschichteter Katheter Stents in die linke Vorderwand- oder Seitenwand-Koronararterie implantiert, wobei die Gefäße zur Stimulation der Restenose durch Gewebehyperplastie leicht überdehnt wurden. Nach 5 Wochen wurden die Tiere reangiographiert und das Maß der Gefäßverengung auf den Angiogrammen mit einem automatischen Computerprogramm gemessen.

| **Gruppe** | **Stenose (%)** |
|---|---|
| Unbeschichtet | 50.49 |
| AcL | 20.22 |
| EAH | 36.01 |
| AcH | 0.86 |
| P | .004 |

Quantitative Koronarangiographie 5 Wochen nach Stentimplantation mit unbeschichteten Kathetern; Stenose = prozentuale Verminderung des Lumendurchmessers im Bereich des Stents im Vergleich zu dem Lumendurchmesser unmittelbar nach Stentimplantation Mittelwert und statistische Signifikanz des Behandlungseffekts.

### Beispiel 8:

Wirkstoffgehalt der Katheter nach Gefäßdilatation und Stentimplantation

Die Ballone aus Beispiel 8 wurden nach Stentimplantation und Entnahme aus den Tieren auf ca. 3 cm Länge von den Ballonkathetern abgetrennt und in 1.5 ml Ethanol überführt. Der Paclitaxelgehalt wurde mittels HPLC bestimmt. Alle verfügbaren beschichteten Ballone und eine Auswahl unbeschichteter Ballone wurden untersucht.

Koronar,
3.0 x 20 mm, Beschichtung: Ac hoch 38 ± 4 µg (n=4)
   Ac niedrig 22 ± 5 µg (n=2)
   EEE hoch 41 (n=1)
3.5 x 20 mm, Beschichtung: Ac hoch 37 ± 10 µg (n=8)
   Ac niedrig 26 ± 6 µg (n=8)
   EEE hoch 53 ± 9 µg (n=9)
Unbeschichtet (unabhängig von Größe und Gefäßgebiet)
   0.9 ± 1.0 µg (n=7)

Aus Beispiel 6 ergibt sich, dass maximal 10 % der Dosis verloren gehen bevor der Ballon expandiert wird und ca. 10 % der Dosis auf dem Ballon verbleiben.

### Beispiel 9:

Probucol wird in einer Konzentration von 100 mg/ml in Aceton eingebracht; die Lösung wird wie in den vorhergehenden Beispielen beschrieben zum Beschichten der Ballonkatheter eingesetzt.

### Beispiel 10:

Rapamycin wird in einer Konzentration von 10 mg/ml in Diethylether gelöst. Die Beschichtung der Ballonanteile der Katheter erfolgt wie in den vorhergehenden Beispielen beschrieben; die Ballone sollen nach der Entnahme aus der Beschichtungslösung möglichst sofort horizontal ausgerichtet und ständig um ihre Längsachse gedreht werden.

### Beispiel 11:

Epothilon B wird in einer Konzentration von 2 mg/ml in Ethylacetat gelöst; die Lösung wird wie in den vorhergehenden Beispielen beschrieben zum Beschichten der Ballonkatheter eingesetzt.

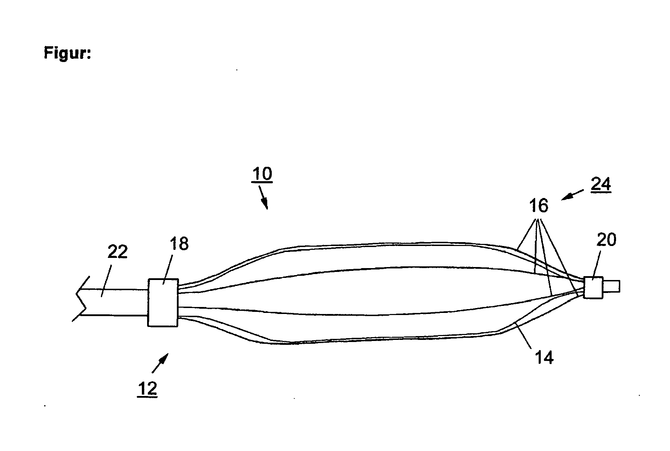

Nachfolgend wird eine in der Figur schematisch dargestellte Ausführungsform der Erfindung am Beispiel einer konkreten Ausgestaltung der medizinischen Vorrichtung 10 dargestellt. Die Vorrichtung 10 besteht dabei aus einem Ballonkatheter 12 mit einem Katheter 22 und einem Ballon 14 sowie eine aus dem Ballon 14 herausragende Vorrichtung 24 zur Schlitzung von Stenosen zumindest im Bereich der erkrankten Gewebsabschnitte oder Organteile aufweist. Die Vorrichtung 24 zur Schlitzung von Stenosen besteht dabei gemäß dem dargestellten Ausführungsbeispiel aus sechs drahtartigen Vorrichtungen 18, die eine gitterartige Konstruktion bilden. Die Längsachse der gitterartigen Konstruktion ist dabei achsparallel zur Längsachse des Ballons 14. Die gitterartige Konstruktion wird an ihren beiden Enden jeweils von einem ersten und zweiten Verbindungselement 18, 20 gehalten. Die Verbindungselemente 18, 20 halten dabei nicht nur die drahtartigen Vorrichtungen 18, sondern dienen auch zur Befestigung der Gitterkonstruktion am Katheter 22. Weitere Ausführungsformen mit einer oder mehreren drahtartigen Vorrichtungen 16 sind denkbar.

Es ist aber auch möglich, dass die Vorrichtung zur Schlitzung von Stenosen aus mindestens einer klingenartigen Vorrichtung oder aus mindestens einem aus dem Ballon herausragenden oder auf der Oberfläche des Ballons aufsitzenden Vorsprung besteht (nicht dargestellt).

Als Materialen für die Herstellung der klingenartige Vorrichtung 16 eignen sich biokompatible Metalle wie zum Beispiel Edelstahl, Metall-Legierungen, Kunststoffe oder Kombinationen davon. Insbesondere können auch sogenannte Formgedächtnislegierungen wie zum Beispiel Nitinol Verwendung finden.

## Patentansprüche

1. Medizinische Vorrichtung zur Arzneimittelabgabe, welche einen Ballonkatheter (12) mit einem Ballon (14) umfasst, wobei an der Oberfläche des Ballons (14) ein lipophiler, weitgehend wasserunlöslicher, an beliebige Gewebebestandteile bindender Arzneistoff mit nach Gewebekontakt sofortiger Wirkstofffreigabe haftet, wobei der Ballonkatheter (12) weiterhin mindestens eine aus dem Ballon (14) herausragende oder auf der Oberfläche des Ballons (14) aufsitzende Vorrichtung (24) zur Schlitzung von Stenosen zumindest im Bereich der erkrankten Gewebsabschnitte oder Organteile aufweist.

2. Vorrichtung nach Anspruch 1, wobei die Vorrichtung (24) zur Schlitzung von Stenosen aus mindestens einer drahtartigen Vorrichtung (16) besteht, wobei die drahtartige Vorrichtung (16) parallel zur Längsachse des Ballons (14) angeordnet ist.

3. Vorrichtung nach Anspruch 1, wobei die Vorrichtung (24) aus mindestens zwei drahtartigen Vorrichtungen (16) aufgebaut ist und eine gitterartige Konstruktion bildet, wobei die Längsachse der gitterartigen Konstruktion parallel oder achsparallel zur Längsachse des Ballons (14) angeordnet ist.

4. Vorrichtung nach Anspruch 2 oder 3, wobei die drahtartige Vorrichtung (16) aus Metall, aus einer Metall-Legierung oder aus Kunststoff besteht.

5. Vorrichtung nach Anspruch 4, wobei die Metall-Legierung eine Formgedächtnislegierung ist.

6. Vorrichtung nach Anspruch 1, wobei die Vorrichtung (24) zur Schlitzung von Stenosen aus mindestens einer klingenartigen Vorrichtung besteht.

7. Vorrichtung nach Anspruch 1, wobei die Vorrichtung (24) zur Schlitzung von Stenosen aus mindestens einem aus dem Ballon (14) herausragenden oder auf der Oberfläche des Ballons (14) aufsitzenden Vorsprung besteht.

8. Vorrichtung nach Anspruch 6 oder 7, wobei die klingenartige Vorrichtung oder der Vorsprung aus Metall, aus einer Metall-Legierung oder aus Kunststoff besteht.

9. Vorrichtung nach einem der vorhergehenden Ansprüche, wobei der Ballonkatheter (12) in Verbindung mit Stents, Katheter und/oder Teile von diesen, Nadeln und Führungsdrähte sowie Stents vorgesehen ist.

10. Vorrichtung nach einem der vorhergehenden Ansprüche, wobei der Ballon (14) vorgeformte Längsfalten aufweist, deren Neigung zur Rückfaltung durch ein Aufdehnen nicht verloren geht.

11. Vorrichtung nach einem der Ansprüche 1 bis 9, wobei die Oberfläche des Ballons (14) glatt ist.

12. Vorrichtung nach einem der Ansprüche 1 bis 9, wobei der Ballon (14) ein in fertig gefaltetem Zustand durch Tauchen in einer niedrig viskosen Wirkstofflösung beschichteter Ballon ist.

13. Vorrichtung nach einem der Ansprüche 1 bis 12, wobei nur ein von Falten abgedeckter Bereich des Ballons (14) mit dem nach des Auftragen getrockneten Arzneistoff bedeckt ist.

14. Vorrichtung nach Anspruch 1, wobei der lipophile Arzneistoff Substanzen zur Hemmung der Zellproliferation oder entzündlicher Prozesse oder Antioxidation umfasst.

15. Vorrichtung nach Anspruch 14, wobei der Arzneistoff Paclitaxel und andere Taxane, Rapamycin und verwandte Substanzen, Tacrolismus und verwandte Substanzen, Corticoide, Sexualhormone und verwandte Substanzen, Statine, Epothilone, Probucol, Prostacycline und/oder Angiogeneseinduktoren umfasst.

16. Vorrichtung nach Anspruch 14 oder 15, wobei der lipophile Arzneistoff als trockene Festsubstanz oder Öl auf der Oberfläche des Ballons (14) vorliegt.

17. Vorrichtung nach Anspruch 16, wobei die wirksame Dosis des Arzneistoffs amorphe Strukturen in einer Partikelgröße zwischen < 0, 1 µm und 5 µm umfassten, die wegen ihrer großen Oberfläche trotz geringer Wasserlöslichkeit der Wirkstoffe schnell in Lösung gehen.

18. Vorrichtung nach Anspruch 1, wobei der lipophile Arzneistoff zur Erzielung einer guten Haftwirkung an der Oberfläche der Vorrichtung und zur Verbesserung der Aufnahme in das Gewebe in eine leicht wasserlösliche Matrixsubstanz eingebettet ist.

19. Vorrichtung nach Anspruch 18, wobei die Matrixsubstanz aus einem niedermolekularen hydrophilen Stoff mit einem Molekulargewicht < 5000 D besteht.

20. Vorrichtung nach Anspruch 1, wobei der lipophile Arzneistoff an Partikel absorbiert oder mit niedermolekularer Matrix auf die Oberfläche des Ballons (14) aufgebracht ist.

21. Vorrichtung nach Anspruch 1, wobei die Oberfläche des Ballons (14) zusätzlich mit Substanzen zur Beeinflussung bestimmter Eigenschaften, wie der Gleitfähigkeit der Vorrichtung oder der Verringerung der Blutgerinnung, beschichtet ist.

22. Verfahren zur Herstellung der Vorrichtung nach Anspruch 1 bis 21, wobei der lipophile Arzneistoff und gegebenenfalls Hilfsstoffe in einem Lösungs-, Suspendier- oder Emulsionsmedium durch Tauchen, Streichen, Sprühen oder mittels Volumenmesseinrichtung auf die Oberfläche des Ballons (14) aufgebracht werden, wobei überschüssige Medien und lose an der Oberfläche haftende Substanzen entfernt werden.

23. Verfahren nach Anspruch 22, wobei der Beschichtungsvorgang bei reproduzierbarer Erhöhung des Wirkstoffgehalts mit gleichen oder verschiedenen Lösungs-, Suspendier- oder Emulsionsmedien und/oder Hilfsstoffen mehrfach durchgeführt wird.

24. Verfahren nach Anspruch 23, wobei als Lösungs-, Suspendier- und Emulsionsmedien Ethanol, Isopropanol, Ethylacetat, Diethylether, Aceton, Dimethylsulfoxid, Dimethylformamid, Glycerin, Wasser oder Mischungen derselben eingesetzt werden.

25. Verfahren nach einem der Ansprüche 22 bis 24, wobei bei gefaltetem Ballon (14) nach der Beschichtung ein Stent auf den Ballonkatheter geschoben und auf diesem festgepresst wird.

26. Verfahren nach Anspruch 25, wobei der Ballon (14) in entfaltetem Zustand mit dem betreffenden lipophilen Arzneistoff beschichtet wird und das Falten des Ballons (14) mit besonders gleitfähigem, gegebenenfalls mit biokompatiblen Gleitmedien benetztem Werkzeug durchgeführt wird.

27. Verfahren nach Anspruch 22, wobei ein Stent vor oder nach dem Beschichtungsvorgang auf den Ballonkatheter montiert wird.

28. Verfahren nach Anspruch 22, wobei der fertig beschichtete Ballonkatheter (14) mittels Ethylenoxid sterilisiert wird.

29. Verwendung der gemäß den Ansprüchen 1 bis 28 ausgebildeten und hergestellten medizinischen Vorrichtung zur Herstellung eines Mittels zur Behandlung von Gefäßerkrankungen oder Durchblutungsstörungen.

30. Verwendung der gemäß den Ansprüchen 1 bis 28 ausgebildeten und hergestellten medizinischen Vorrichtung zur Herstellung eines Mittels zur Schaffung von offenen Passagen im Körper.

## Claims

1. Medical device for drug release comprising a balloon catheter (12) with a balloon (14), wherein a lipophilic, largely water-insoluble drug that binds to any tissue components and immediately releases the active agent when in contact with tissue adheres to the surface of the balloon (14),
wherein the balloon catheter (12) further includes at least one device (24) for slitting stenosis at least in the region of the diseased tissue or organ parts, that protrudes from or is located on the surface of the balloon (14).

2. Device according to claim 1, wherein the device (24) for slitting stenosis consists of at least one wire-like device (16), wherein the wire-like device (16) is positioned in parallel to the longitudinal axis of the balloon (14).

3. Device according to claim 1, wherein the device (24) is constructed of at least two wire-like devices (16) and forms a grid-like construction, wherein the longitudinal axis of the grid-like construction is positioned in parallel or axially parallel to the longitudinal axis of the balloon (14).

4. Device according to claim 2 or 3, wherein the wire-like device (16) consists of metal, a metal alloy or of plastic.

5. Device according to claim 4, wherein the metal alloy is a shape memory alloy.

6. Device according to claim 1, wherein the device (24) for slitting stenosis consists of at least one blade-like device.

7. Device according to claim 1, wherein the device (24) for slitting stenosis consists of at least one protrusion that protrudes from the balloon (14) or is positioned on the surface of the balloon (14).

8. Device according to claim 6 or 7, wherein the blade-like device or the protrusion consists of metal, a metal alloy or of plastic.

9. Device according to any of the preceding claims, wherein the balloon catheter (12) is intended for conjunction with stents, catheters and/or parts thereof, needles and guide wires as well as stents.

10. Device according to any of the preceding claims, wherein the balloon (14) has preformed longitudinal folds, whose tendency to fold back is not lost by an expansion.

11. Device according to any of claims 1 to 9, wherein the surface of the balloon (14) is smooth.

12. Device according to any of claims 1 to 9, wherein the balloon (14) is a balloon that has been coated in the readily folded state by dipping in a low viscous drug solution.

13. Device according to any of claims 1 to 12, wherein only an area covered by folds of the balloon (14) is covered by the drug dried after application.

14. Device according to claim 1, wherein the lipophilic drug comprises substances for inhibiting cell proliferation or inflammatory processes, or antioxidants.

15. Device according to claim 14, wherein the drug comprises paclitaxel and other taxanes, rapamycin and related substances, tacrolismus and related substances, corticoide, sexual hormones and related substances, statins, epothilones, probucol, prostacyclines and/or angiogenesis inducers.

16. Device according to claim 14 or 15, wherein the lipophilic drug is present as a dry solid substance or as an oil on the surface of the balloon (14).

17. Device according to claim 16, wherein the effective doses of the drug comprises amorphous structures having a particle size between <0.1 µm and 5 µm, which, despite the lower water solubility of the drugs, dissolve fast because of their large surface.

18. Device according to claim 1, wherein the lipophilic drug is embedded in an easily water soluble matrix substance to achieve a good adherence at the surface of the device and for improving the uptake into the tissue.

19. Device according to claim 18, wherein the matrix substance consists of a low molecular hydrophilic substance with a molecular weight of < 5000 D.

20. Device according to claim 1, wherein the lipophilic drug is applied to the surface of the balloon (14) being absorbed to particles or with a low molecular matrix.

21. Device according to claim 1, wherein the surface of the balloon (14) is additionally coated with substances for influencing particular properties, such as friction of the device or the lowering of blood clotting.

22. Process for the manufacture of a device according to any of claims 1 to 21, wherein the lipophilic drug and possibly excipients are applied to the surface of the balloon (14) in a solvent, suspension or emulsion medium by dipping, painting or spraying or using a volume measuring device, wherein excessive medium and substances loosely adhering to the surface will be removed.

23. Process according to claim 22, wherein the coating process is repeatedly conducted with a reproducible increase of the drug content with the same or different solution, suspension or emulsion media and/or excipients.

24. Process according to claim 23, wherein ethanol, isopropanol, ethyl acetate, diethyl ether, acetone, dimethyl sulfoxide, dimethyl formamide, glycerin, water or mixtures thereof are used as solution, suspension and emulsion medium.

25. Process according to any of claims 22 to 24, wherein, after the coating, a stent is advanced over the catheter balloon with the balloon in its folded state and is crimped thereon.

26. Process according to claim 25, wherein the balloon (14) is coated with the respective lipophilic drug in the unfolded state, and wherein the folding of the balloon (14) is conducted with a particularly slidable tool, possibly wetted with a biocompatible friction media.

27. Process according to claim 22, wherein a stent is mounted to the balloon catheter before or after the coating process.

28. Process according to claim 22, wherein the readily coated balloon catheter (14) is sterilized by ethylene oxide.

29. Use of the medical device designed or manufactured according to claims 1 to 28 for manufacturing a means for treating vasculature diseases or circulatory disorders.

30. Use of the medical device designed or manufactured according to claims 1 to 28 for manufacturing a means for creating patent passages in the body.

## Revendications

1. Dispositif médical pour l'administration de médicaments, qui comprend un cathéter à ballon (12) avec un ballon (14), dans lequel un médicament lipophile, largement insoluble dans l'eau, se liant à des composants quelconques d'un tissu, adhère à la surface du ballon (14), avec une libération du principe actif immédiat après le contact avec le tissu, le cathéter à ballon (12) comprenant en outre au moins un dispositif dépassant du ballon (24) ou reposant sur la surface du ballon (14), pour l'incision de sténoses au moins au niveau des portions de tissus ou des parties d'organes malades.

2. Dispositif selon la revendication 1, dans lequel le dispositif (24) pour l'incision de sténoses est constitué d'au moins un dispositif filaire (16), le dispositif filaire (16) étant disposé parallèlement à l'axe longitudinal du ballon (14).

3. Dispositif selon la revendication 1, dans lequel le dispositif (24) est constitué d'au moins deux dispositifs filaires (16) et constitue d'une structure en forme de grille, l'axe longitudinal de la structure en forme de grille étant disposé parallèlement ou axialement par rapport à l'axe longitudinal du ballon (14).

4. Dispositif selon la revendication 2 ou 3, dans lequel le dispositif filaire (16) est constitué de métal, d'un alliage métallique ou de matière plastique.

5. Dispositif selon la revendication 4, dans lequel l'alliage métallique est un alliage à mémoire de forme.

6. Dispositif selon la revendication 1, dans lequel le dispositif (24) pour l'incision de sténoses est constitué d'au moins un dispositif en forme de lame.

7. Dispositif selon la revendication 1, dans lequel le dispositif (24) pour l'incision de sténoses est constitué d'au moins une saillie dépassant du ballon (14) ou reposant sur la surface du ballon (14).

8. Dispositif selon la revendication 6 ou 7, dans lequel le dispositif en forme de lame ou la saillie est constitué d'un métal, d'un alliage métallique ou de matière plastique.

9. Dispositif selon l'une des revendications précédentes, dans lequel le cathéter à ballon (12) est prévu en lien avec des stents, des cathéters et/ou de parties de ceux-ci, des aiguilles et des fils de guidage ainsi que des stents.

10. Dispositif selon l'une des revendications précédentes, dans lequel le ballon (14) présente des plis longitudinaux pré-formés dont l'inclinaison pour le repliage n'est pas perdue lors d'une dilatation.

11. Dispositif selon l'une des revendications 1 à 9, dans lequel la surface du ballon (14) est lisse.

12. Dispositif selon l'une des revendications 1 à 9, dans lequel le ballon (14) est un ballon revêtu par immersion dans un état entièrement plié dans une solution de principe actif de faible viscosité.

13. Dispositif selon l'une des revendications 1 à 12, dans lequel seule une zone du ballon (14), recouverte par le pliage, est recouverte par le médicament séché après l'application.

14. Dispositif selon la revendication 1, dans lequel le médicament lipophile comprend des substances permettant d'empêcher la prolifération cellulaire, des processus inflammatoires ou une anti-oxydation.

15. Dispositif selon la revendication 14, dans lequel le médicament comprend le paclitaxel, et autres taxanes, la rapamycine et substances apparentées, le tacrolismus et substances apparentées, les corticoïdes, les hormones sexuelles et substances apparentées, les statines, les épothilones, le probucol, les prostacyclines et/ou les inducteurs d'angiogenèse.

16. Dispositif selon la revendication 14 ou 15, dans lequel le médicament lipophile est sous la forme d'une substance solide sèche ou d'une huile sur la surface du ballon (14).

17. Dispositif selon la revendication 16, dans lequel la dose efficace du médicament comprend des structures amorphes dans une taille de particules entre <0,1 µm et 5 µm, qui sont dissoutes rapidement, du fait de leur surface importante, malgré la faible solubilité des principes actifs dans l'eau.

18. Dispositif selon la revendication 1, dans lequel le médicament lipophile est intégré, afin d'obtenir une bonne adhésion à la surface du dispositif et d'améliorer l'absorption dans le tissu, dans une substance matricielle facilement soluble dans l'eau.

19. Dispositif selon la revendication 18, dans lequel la substance matricielle est constituée d'un matériau hydrophile de faible poids moléculaire avec un poids moléculaire < 5000 D.

20. Dispositif selon la revendication 1, dans lequel le médicament lipophile est appliqué de manière absorbée sur des particules ou avec une matrice de faible poids moléculaire à la surface sur ballon (14).

21. Dispositif selon la revendication 1, dans lequel la surface du ballon (14) est revêtue en outre par des substances permettant de contrôler certaines propriétés comme la capacité de glissement du dispositif ou la réduction de la coagulation.

22. Procédé pour la fabrication du dispositif selon la revendication 1 à 21, dans lequel le médicament lipophile et, le cas échéant, des substances auxiliaires, sont appliqués, dans un milieu de solution, de suspension ou d'émulsion, par immersion, application par pinceau, pulvérisation ou au moyen d'un système de mesure de volume sur la surface du ballon (14), les milieux en excès et les substances adhérant de manière lâche à la surface étant éliminés.

23. Procédé selon la revendication 22, dans lequel le processus de revêtement est effectué plusieurs fois dans le cas d'une augmentation reproductible de la teneur en principe actif avec des milieux de solution, de suspension ou d'émulsion et/ou des substances auxiliaires identiques ou différents.

24. Procédé selon la revendication 23, dans lequel de l'éthanol, de l'isopropanol, de l'acétate d'éthyle, du diéthyléther, de l'acétone, du diméthylsulfoxyde, du diméthylformamide, de la glycérine, de l'eau ou des mélanges de ceux-ci sont utilisés en tant que milieux de solution, de suspension et d'émulsion.

25. Procédé selon l'une des revendications 22 à 24, dans lequel un stent est poussé sur le cathéter à ballon et comprimé sur celui-ci après le revêtement, le ballon (14) étant plié.

26. Procédé selon la revendication 25, dans lequel le ballon (14) dans l'état déplié est revêtu avec le médicament lipophile concerné et le pliage du ballon (14) est effectué avec un outil imprégné, le cas échéant, avec des substances de glissement biocompatibles.

27. Procédé selon la revendication 22, dans lequel un stent est monté sur le cathéter à ballon avant ou après le processus de revêtement.

28. Procédé selon la revendication 22, dans lequel le cathéter à ballon (14) une fois revêtu est stérilisé avec de l'oxyde d'éthylène.

29. Utilisation du dispositif médical conçu et fabriqué selon les revendications 1 à 28, pour la fabrication d'un moyen pour le traitement de maladies vasculaires ou de troubles circulatoires.

30. Utilisation du dispositif médical conçu et fabriqué selon les revendications 1 à 28, pour la fabrication d'un moyen pour la réalisation de passages ouverts dans le corps.
